# EUROPÄISCHE PATENTSCHRIFT

(11) **EP 1 161 934 B1**
(45) Veröffentlichungstag und Bekanntmachung des Hinweises auf die Patenterteilung: **28.04.2004**
(21) Anmeldenummer: 01112640.6
(22) Anmeldetag: 25.05.2001
(51) Int. Cl.: A61K 7/11

(54) **Haarspray**
Hairspray
Laque capillaire en aérosol

(30) Priorität: 08.06.2000 DE 10028524
(43) Veröffentlichungstag der Anmeldung: 12.12.2001
(73) Patentinhaber: KPSS-Kao Professional Salon Services GmbH, 64297 Darmstadt (DE)
(72) Erfinder: Link, Thorsten, Dr., 64319 Pfungstadt (DE); Weichaus, Dirk, 64404 Bickenbach (DE)

(56) Entgegenhaltungen:
- WO-A-00/06092
- WO-A-96/00564
- WO-A-98/52517
- FR-A- 2 462 941
- US-A- 3 730 182
- US-A- 5 985 294

## Beschreibung

Die vorliegende Erfindung betrifft ein Haarspray mit verbesserten Gebrauchseigenschaften, das insbesondere eine gezielte Applikation am Haar, d.h. eine geringe Vernebelung, und damit einen effektiveren Einsatz des Filmbildners ermöglicht und gleichzeitig eine Zusammensetzung mit einem niedrigen Gehalt an leichtflüchtigen Bestandteilen enthält, also als "Low VOC-Produkt", konzipiert werden kann.

Haarsprays mit einem niedrigen Gehalt an leichtflüchtigen Bestandteilen sind bekanntermaßen erwünscht, da sie als umweltfreundlich gelten.
Ihrem Gehalt an weniger flüchtigen Substanzen sind jedoch natürliche Grenzen gesetzt, da die damit erhaltenen Sprays "naß" sind und damit auch keine ausreichenden haarpflegenden Eigenschaften bei befriedigenden Trocknungszeiten verleihen.
Überdies lassen sich diese Haarsprays in Aerosoldosen mit üblichen Sprühdüsen nicht gezielt auf spezielle Teilflächen des Haares richten, was vom Verbraucher insbesondere zum Frisurenaufbau aber als erwünscht angesehen wird.

Es bestand daher ein Bedürfnis, Haarsprays zu entwickeln, die diese Anforderungen zuverlässig erfüllen.

Die vorliegende Erfindung löst diese Aufgabe durch eine Aerosoldose enthaltend eine Zusammensetzung, die
a) 1 bis 15 Gew.-% mindestens eines Filmbildners;
b) 25 bis 80 Gew.-% mindestens eines Lösungsmittels mit einem Siedepunkt zwischen etwa 30 und 95°C bei Normaldruck;
c) mindestens ein Treibmittel, ausgewählt aus Kohlenwasserstoffen, 1,1-Difluorethan, Dimethylether, Kohlendioxid und/oder Stickstoff; und, gegebenenfalls,
d) Wasser, enthält
deren Sprühdüse mit einem rohrförmigen Fortsatz ausgestattet ist, der einen Öffnungsdurchmesser von 0,2 bis 1,5 mm und eine Länge von 3 bis 100 mm aufweist und einen Sprühstrahl mit einem Austrittsventil von 10° bis 25° abgibt.

Als Filmbildner in den erfindungsgemäßen wäßrigen Haarsprayzusammensetzungen können an sich bekannte alkohol- bzw. wasserlösliche Polymere wie nichtionische Polymere, vorzugsweise ein alkohol- bzw. wasserlösliches Vinylpyrrolidon-Polymer wie ein Vinylpyrrolidon-Homopolymer oder ein -Copolymerisat, insbesondere mit Vinylacetat, verwendet werden.

Geeignete Vinylpyrrolidon-Polymere sind z.B. die unter dem Handelsnamen "Luviskol^{R}" bekannten Produkte, beispielsweise die Homopolymerisate "Luviskol^{R} K 30, K 60 und K 90", sowie die alkohol- bzw. wasserlöslichen Copolymerisate aus Vinylpyrrolidon und Vinylacetat, die unter dem Handelsnamen "Luviskol^{R} VA 55 bzw. VA 64" von der BASF AG vertrieben werden.

Weitere geeignete nichtionische Polymere sind Vinylpyrrolidon/Vinylacetat/Vinylpropionat-Copolymere wie "Luviskol^{R} VAP 343^{R}", Vinylpyrrolidon/(Meth)-Acrylsäureester-Copolymere sowie Chitosan-Derivate.

Ihr Anteil in den erfindungsgemäßen Haarsprays liegt zwischen etwa 1,0 und etwa 15, vorzugsweise 2,5 und 10 Gew.-%, bezogen auf die Gesamtzusammensetzung des Mittels.

Es können jedoch auch andere Polymere (mit-)verwendet werden.

Eine große Variationsbreite besteht bei den kationischen, anionischen und/oder amphoteren Polymeren, die in einer Menge zwischen 1 und 15 Gew.-%, vorzugsweise 1,5 bis 10, insbesondere 2 bis 7,5 Gew.-%, je nach Charakter des Polymeren, allein oder im Gemisch untereinander oder mit nichtionischem Polymer enthalten sein können.

Kationische Polymere sind neben den altbekannten quaternären Cellulosederivaten des Typs "Polymer^{R}JR" insbesondere quaternisierte Homo- Copolymere des Dimethyldiallylammoniumchlorids, wie sie unter dem Handelsnamen "Merquat^{R}" im Handel sind, quaternäre Vinylpyrrolidon-Copolymere, insbesondere mit Dialkylaminoalkyl(meth)acrylaten, wie sie unter dem Namen "Gafquat^{R}" bekannt sind, Copolymerisate aus Vinylpyrrolidon und Vinylimidazoliniummethochlorid, die unter dem Handelsnamen "Luviquat^{R}" angeboten werden, Polyamino-Polyamid-Derivate, beispielsweise Copolymere von Adipinsäure-Dimethylaminohydroxypropyldiethytentriamin, wie sie unter dem Namen "Cartaretine^{R}F" vertrieben werden, sowie auch bisquaternäre langkettige Ammoniumverbindungen der in der US-PS 4 157 388 beschriebenen Harnstoff-Struktur, die unter dem Handelsnamen "Mirapol A 15" im Handel sind.

Geeignete kationische Polymere, insbesondere im Gemisch mit nichtionischen, amphoteren bzw. zwitterionischen und/oder anionischen Polymeren sind auch die aus der EP 640 643 A1 bekannten Organopolysiloxan/Poly-(N-acylalkylenimin)-Copolymerisate.

Verwiesen wird in diesem Zusammenhang auch auf die in den DE-Osen 25 21 960, 28 11 010, 30 44 738 und 32 17 059 genannten kationaktiven Polymeren sowie die in der EP-A 337 354 auf den Seiten 3 bis 7 beschriebenen Produkte.
Es können auch Mischungen aus verschiedenen kationischen Polymeren eingesetzt werden. Der bevorzugte Anteil eines solchen kationischen Polymeren liegt bei 0,5 bis 10, vorzugsweise 1 bis 7,5, insbesondere 1,5 bis 5 Gew.-% des Mittels.

Als amphotere Polymere, die zum Einsatz gelangen können, seien insbesondere Copolymerisate aus N-Octylacrylamid, (Meth)Acrylsäure und tert.-Butylaminoethylmethacrylat vom Typ "Amphomer^{R}", Copolymerisate aus Methacryloylethylbetain und Alkylmethacrylaten vom Typ "Yukaformer^{R}", z.B. das Butylmethacrylat-Copolymere "Yukaformer^{R} Am75"; Copolymerisate aus Carboxylgruppen und Sulfongruppen enthaltenden Monomeren, z.B. (Meth)Acrylsäure und Itaconsäure, mit basischen Gruppen, insbesondere Aminogruppen enthaltenden Monomeren wie Monp- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US-A 3,927,199 bekannten Copolymeren genannt.

Die Menge entspricht in etwa derjenigen des nichtionischen Polymers.

Besonders geeignete anionische Polymere, deren Menge vorzugsweise bei etwa 1 bis etwa 10, insbesondere etwa 2,5 bis etwa 7,5 Gew.-% der Gesamtzusammensetzung des Mittels liegt, sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez^{R} AN oder ES" vertrieben werden. Diese Polymeren können auch teilverestert sein, beispielsweise "Gantrez^{R} ES 225", der Ethylester eines Ethylvinylethers/Maleinsäure-Copolymers, oder der Butyl- oder Isobutylester desselben.

Weitere geeignete anionische Polymere sind insbesondere Vinylacetat/Crotonsäureoder Vinylacetat/Vinylneodecanoat/Crotonsäure-Copolymere des Typs "Resyn", Natriumacrylat/Vinylalkohol-Copolymere des Typs "Hydagen^{R}F", Natriumpolystyrolsulfonat, z.B. Flexan^{R} 130"; Ethylacrylat/Acrylsäure/N-tert.-Butylacrylamid-Copolymere des Typs "Ultrahold^{R}", Vinylpyrrolidon/Vinylacetat/Itaconsäure-Copolymere, Acrylsäure/Acrylamid-Copolymere bzw. Natriumsalze derselben vom Typ. "Reten^{R}"; etc.

Sie können erforderlichenfalls mit Ammoniak, Natrium- bzw. Kaliumhydroxid und/oder Aminen ganz oder teilweise neutralisiert sein.

Polymergemische können im Rahmen ihrer Verträglichkeit verwendet werden.

Als Lösungsmittel mit einem Siedepunkt zwischen etwa 30 und etwa 95°C bei Normaldruck in den erfindungsgemäßen Zusammensetzungen sind vor allem Ethanol, n-Propanol, Isopropylalkohol und gesättigte Kohlenwasserstoffe, insbesondere n-Pentan, vorhanden.

Ihr Anteil beträgt 25 bis 80, insbesondere zwischen etwa 35 und 70 Gew.-%, berechnet auf die Gesamtzusammensetzung.

Der Treibmittelanteil ist in der Regel so eingestellt, daß ein Druck zwischen etwa 1,5 und etwa 8 bar, insbesondere etwa 2,5 und 6 bar, bei 20°C in der Dose erhalten wird.

Bevorzugtes Treibmittel ist Dimethylether, insbesondere in einer Menge von etwa 20 bis 40 Gew.-%, berechnet auf die Gesamtzusammensetzung.
Es sind jedoch auch Propan, n-Butan und Isobutan bzw. deren handelsübliche Gemische und 1,1-Difluorethan sowie komprimierte Treibgase wie Kohlendioxid und Stickstoff geeignet.

Selbstverständlich können in den erfindungsgemäßen Zusammensetzungen alle in Haarsprays üblichen und bekannten Stoffe mitverwendet werden, vorausgesetzt, daß sie mit den essentiellen Wirkstoffen nicht interferieren. Solche Stoffe sind beispielsweise oberflächenaktive Mittel, Weichmacher, Verdickungsmittel, Farbstoffe, Riechstoffe, Konservierungsmittel, etc.

Bei der erfindungsgemäße eingesetzten Aerosoldose kann es sich um eine Monoblock- oder Weißblechdose handeln.

Die Verlängerung der Sprühdüse besteht aus einem etwa 3 bis 100 mm, insbesondere 10 bis etwa 50 mm langem Rohr, dessen Öffnung einen Durchmesser von 0,2 bis 1,5, insbesondere etwa 0,3 bis 1 mm, aufweist.

Dadurch ist es möglich, einen Austrittswinkel des Sprühstrahls zwischen etwa 10 und 25° zu erzielen, der eine punktuelle Ausrichtung auf bestimmte zu besprühende Teile des Haares bzw. der Frisur ermöglicht.

Die folgenden Beispiele illustrieren die Erfindung.

### Beispiel 1

Eine Haarspray-Zusammensetzung, bestehend aus

| | |
|---|---|
| Vinylpyrrolidon/Vinylacetat-Copolymer (Luviskol^{R} 37 I) | 30 (Gew.-%) |
| Ethanol | 68,5 |
| Polysilicone-9 | 1,7 |
| Parfum | 0,3 |

wurde mit einem Gemisch aus

| | |
|---|---|
| n-Pentan | 45 (Gew.-%) |
| Dimethylether | 55 |

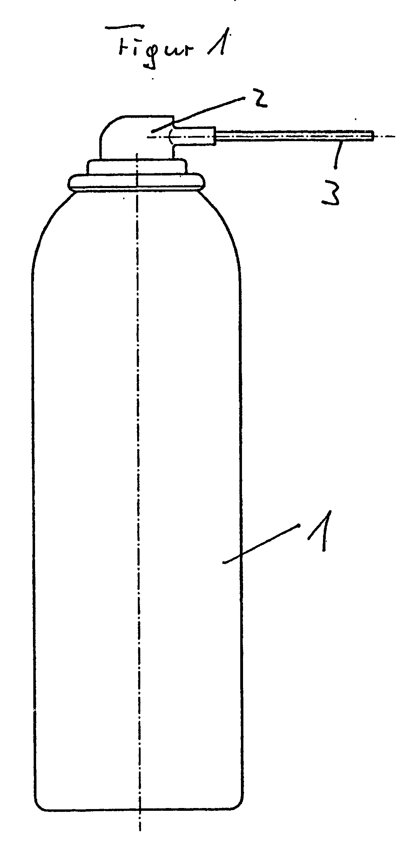

im Gewichtsverhältnis von 35:65 in eine Aluminium-Monoblock-Aerosoldose (1) nach Figur 1 abgefüllt, die an ihrer Sprühdüse (2) eine 45 mm lange rohrförmige Verlängerung (3) mit einem Öffnungsdurchmesser von 0,8 mm aufwies.

Durch diese Anordnung wurde ein Sprühstrahl mit einem Austrittswinkel von 15 bis 18° erhalten, der damit zielgerichtet eine Haarfestigung an der gewünschten Stelle erreichte und daher auch vorzüglich zum Frisuraufbau geeignet war.

### Beispiel 2

Eine Haarspray-Zusammensetzung aus

| | |
|---|---|
| Polysilicon-9 | 1,0 (Gew.-% |
| N-Octylacrylamid/tert.-Butylaminoethylmethacrylat/Acrylsäure-Copolymerisat (Amphomer^{R}) | 5,0 |
| 2-Amino-2-methyl-1-propanol | 1,0 |
| Isopropylalkohol | 35,0 |
| PEG-40-Hydriertes Ricinusöl | 0,1 |
| Parfum | 0,2 |
| Dimethylether | 33,0 |
| Wasser | ad 100,0 |

wurde in eine Weißblechdose (1) abgefüllt, die mit einer einen 50 mm langen rohrförmigen Fortsatz (3) aufweisenden Sprühdüse (2) ausgestattet war, der einen Öffnungsdurchmesser von etwa 1 mm aufwies.

## Patentansprüche

1. Aerosoldose enthaltend eine Haarspray-Zusammensetzung, die,
a- 1 bis 15 Gew.-% mindestens eines Filmbildners;
b- 25 bis 80 Gew.-% mindestens eines Lösungsmittels mit einem Siedepunkt zwischen 30 und 95°C bei Normaldruck;
c- mindestens ein Treibmittel, ausgewählt aus Kohlenwasserstoffen, 1,2-Difluorethan, Dimethylether, Kohlendioxide und/oder Stickstoff; und gegebenenfalls,
d- Wasser, enthält,
**dadurch gekennzeichnet, dass** die Sprühdüse der Aerosoldose mit einem rohrförmigen Fortsatz ausgestattet ist, der einem Öffnungsdurchmesser von 0,2 bis 1,5 mm und eine Länge von 3 bis 100 mm aufweist und einen Sprühstrahl mit einem Austrittsventil von 10 bis 25° abgibt.

2. Aerosoldose nach Anspruch 1, **dadurch gekennzeichnet, dass** das Treibmittel C-Dimethylether ist.

3. Aerosoldose nach Anspruch 1 oder 2, **dadurch gekennzeichnet, dass** das Lösungsmittel b- 25 bis 50 Gew.-% n-Pentan, berechnet auf die Gesamtzusammensetzung, entlölt.

## Claims

1. Aerosol can containing a hair spray composition, comprising
a) 1 % to 15 % by weight of at least one film-forming agent;
b) 25 % to 80 % by weight of at least one solvent with a boiling point between about 30 ° and 95°C at normal pressure;
c) at least one propellant, selected from hydrocarbons, 1.2-difluoroethane, dimethyl ether, carbon dioxide, and /or nitrogen, and, optionally,
d) water,
**characterized in that** the spray nozzle of the can is provided with a tube-shaped extension having an opening with a diameter of 0.2 mm to 1.5 mm and a length of 3 mm to 100 mm, dispensing a spray stream with an ejection angle of 10° to 25°.

2. Aerosol can according to claim 1, **characterized in that** the propellant (component c) is dimethyl ether.

3. Aerosol can according to claim 1 or 2, **characterized in that** it comprises as solvent (component b) 25 % to 50 % by weight of n-pentane, calculated to the total composition.

## Revendications

1. Bombe aérosol contenant une composition de spray capillaire, qui contient
a) de 1 à 15 % en poids d'au moins un agent filmogène ;
b) de 25 à 80 % en poids d'un solvant présentant un point d'ébullition entre 30 et 95 ° C pour une pression normale ;
c) au moins un agent de propulsion, choisi parmi des hydrocarbures, le 1-2 difluoréthane, le diméthyléther, le dioxyde de carbone et/ ou l'azote ; et le cas échéant,
d) de l'eau,
**caractérisée en ce que** la buse de vaporisation de la bombe aérosol est équipée d'un prolongement tubulaire, qui présente un diamètre d'ouverture de 0,2 à 1,5 mm et une longueur de 3 à 100 mm et qui délivre un jet de vaporisation, à l'aide d'une soupape de sortie de 10 à 25 °.

2. Bombe aérosol selon la revendication 1, **caractérisée en ce que** l'agent de propulsion c) est du diméthyléther.

3. Bombe aérosol selon la revendication 1 ou 2, **caractérisée en ce que** le solvant b) contient de 25 à 50 % en poids de n-pentané, calculé sur la composition globale.
